(19) Europäisches Patentamt
European Patent Office
Office européen des brevets

(11) Publication number: **0 040 338**
**A2**

(12) # EUROPEAN PATENT APPLICATION

(21) Application number: 81103279.6

(22) Date of filing: 05.04.79

(51) Int. Cl.³: C 07 C 45/46
C 07 C 49/792, C 11 B 9/00

(30) Priority: 19.04.78 US 897903

(43) Date of publication of application:
25.11.81 Bulletin 81/47

(84) Designated Contracting States:
BE CH DE FR GB IT LU NL SE

(60) Publication number of the earlier application in accordance with Art. 76 EPC: 0 004 914

(71) Applicant: INTERNATIONAL FLAVORS & FRAGRANCES INC.
521 West 57th Street
New York New York 10019(US)

(72) Inventor: Sprecker, Mark A.
6 Sandpiper Lane
Sea Bright New Jersey 07760(US)

(72) Inventor: Theimer, Ernst T.
150 Rumson ROad
Rumson New Jersey 07760(US)

(74) Representative: Brown, John David et al,
FORRESTER & BOEHMERT Widenmayerstrasse 4/I
D-8000 München 22(DE)

(54) **Process for the preparation of acylated indanes and tetrahydronaphthalenes.**

(57) Process for producing compounds useful in perfumery including acylated indanes and tetrahydronaphthalenes using a lower acid derivative in the presence of a hydrocarbon or hydrocarbon mixture containing $C_5$ to $C_{10}$ alkanes at a temperature of at least −20°C; preferably up to −5°C.

EP 0 040 338 A2

TITLE MODIFIED
see front page

"Process for the production of compounds useful in perfumery"

THE PRESENT INVENTION relates to a process for producing synthetic musks such as acylated indanes and acylated tetrahydronaphthalenes using an economical process involving the use of one reactor and one physical step.

Prior to the instant invention, synthetic musks such as 5-acetyl-1,1,2,3,3,6-hexamethylindane having the structure:

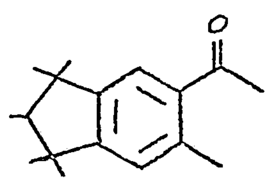

and 6-acetyl-1,1,3,4,4,6-hexamethyltetrahydronaphthalene were produced using several complicated and low-yield processes. Arctander, "Perfume and Flavor Chemicals (aroma chemicals)" published by the author in 1969, volume I, Monographs numbers 40 and 41 discloses such acylated indane musks.

The use of six, seven or eight carbon saturated hydrocarbons as solvents in order to aid Friedel-Crafts catalysis is disclosed at column 3, lines 5-10 of the United States Patent 3,439,056 but the nature of the reaction (conversion of a first type of acylcyclic compound to a second type of acylcyclic compound using a specific catalyst) is different in kind from the reaction of the instant invention.

Thus, the present invention fulfills a need in the perfume industry in that it provides a simplified, economical process for producing synthetic musks such as acylated indanes and acylated tetrahydronaphthalenes using an economical process involving the use of one reactor and one physical

BAD ORIGINAL

step. More specifically, the present invention provides a process for preparing acyl benzene derivatives having the structures:

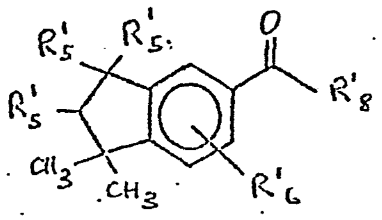

or

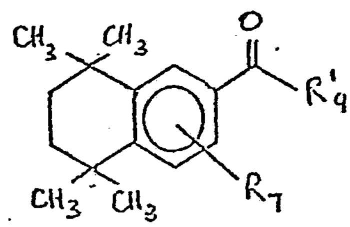

wherein $R'_5$ is hydrogen or methyl; $R_6$ and $R'_7$ are the same or different hydrogen or $C_1$-$C_3$ lower alkyl; $R'_8$ and $R'_9$ are $C_1$ to $C_3$ and are the same or different lower alkyl, comprising the steps of reacting an indane or tetrahydronaphthalene having the structures:

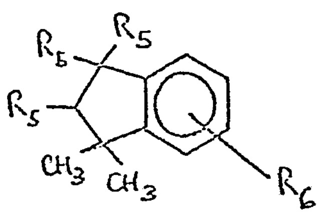

or

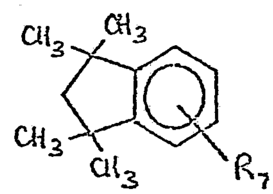

with an acid derivative having the structure:

$$R'_{10}-\overset{O}{\overset{\|}{C}}-X'$$

wherein $R'_{10}$ is $C_1$-$C_3$ lower alkyl and X' is $-O-\overset{O}{\overset{\|}{C}}-R'_{10}$ or chloride, bromide or iodide, in the presence of a saturated hydrocarbon or hydrocarbon mixture comprising the groups $C_5$ to $C_{10}$ alkanes at a temperature between $-20^{o}$ and $-5^{o}C$ in the presence of an aluminium chloride catalyst; the ratio of the aliphatic saturated hydrocarbon to the indane or tetrahydronaphthalene derivative being from 1:10 up to 10:1; the mole ratio of aluminium chloride to acid derivative being 1:2 to 2:1; the mole ratio of indane or tetrahydronaphthalene derivative to acid derivative being from 1:1 up to 10:1.

Advantageously, the ratio of hydrocarbon reactant to acid derivative

BAD ORIGINAL

is 2:1.

In a preferred embodiment the saturated hydrocarbon or hydrocarbon mixture comprises one or more of n-hexane, n-octane and 2, 2, 4-trimethyl pentane.

Preferably the ratio of saturated hydrocarbon to hydrocarbon reactant is from 2:10 to 3:10.

Examples of reactions within the scope of this invention are as follows:

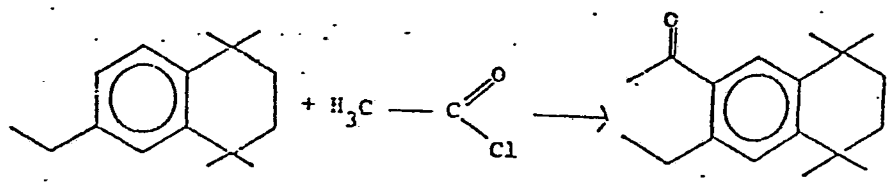

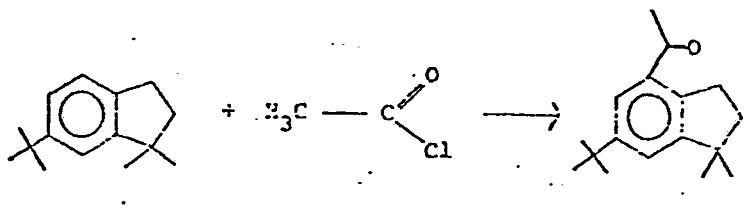

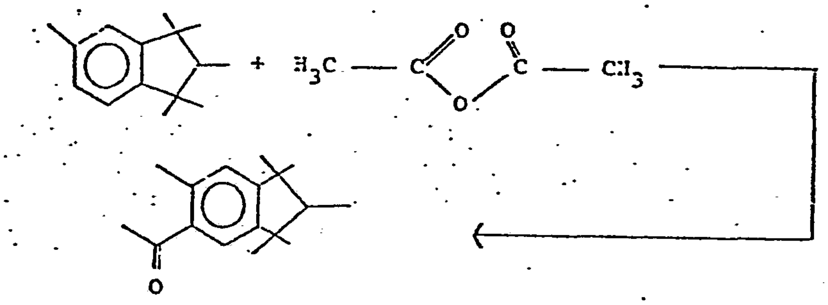

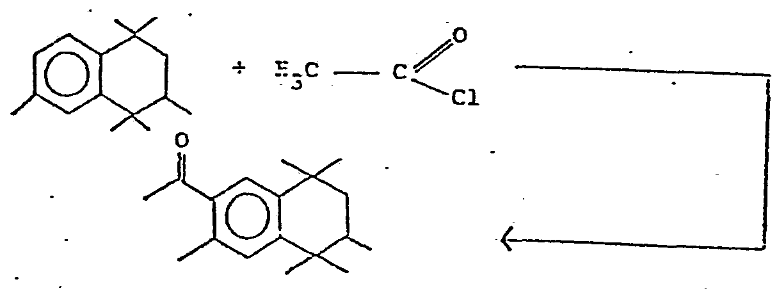

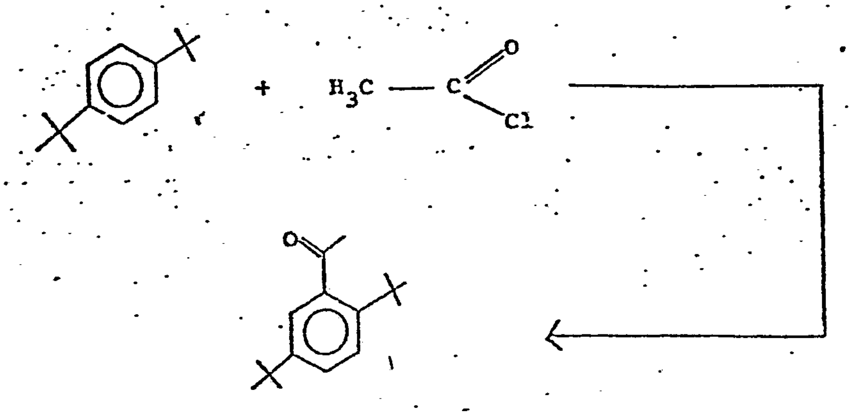

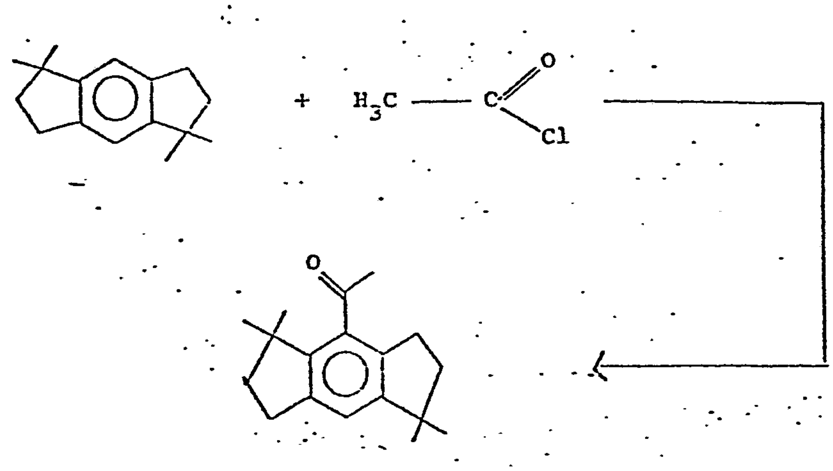

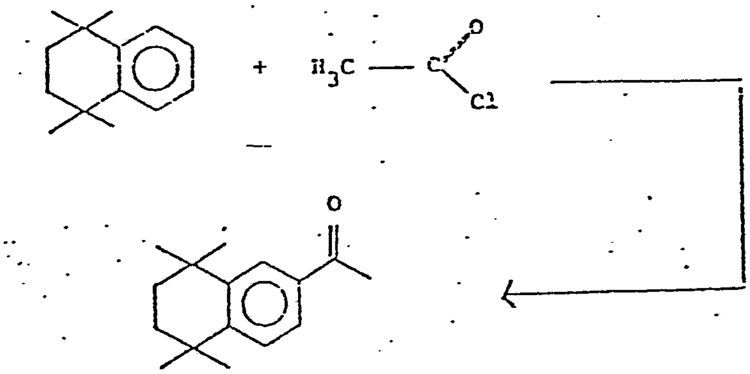

It is the use of the aliphatic saturated hydrocarbon in conjunction with the Friedel-Crafts reactant and the aluminium chloride which causes the reaction mass to decrease in viscosity, thereby improving dispersion and heat transfer. Improved yields of desired product in the foregoing reaction sequences are achieved by minimizing undesirable side reactions and shortening overall reaction times:

The following examples are given to illustrate embodiments of the invention as it is presently preferred to practice. It will be understood that these examples are illustrative and the invention is not to be considered as restricted thereto except as indicated in the appended claims.

Example I

4-Acetyl-6-t-butyl-1, 1-dimethylindane

Reaction:

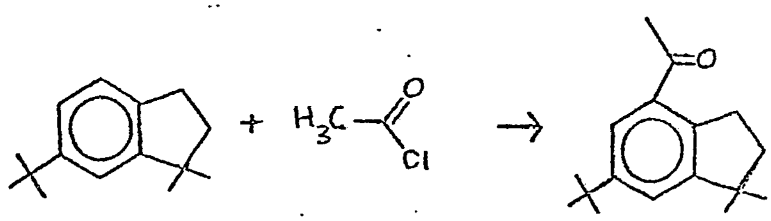

A solution of 1, 1-dimethyl-6-t-butylindane (600 grams) and acetyl chloride (314 grams) is added over a 4 hour period at -10°C to a slurry of aluminium chloride (534 grams), 1, 1-dimethyl-6-t-butylindane (1420 grams) and n-hexane (400 grams). The resulting reaction mass is poured into 5 litres of ice water with vigorous stirring thereby forming 2 liquid layers. The bottom (aqueous) layer is discarded and the organic layer is washed twice with 2 litres of water. Distillation affords recovered 1, 1-dimethyl-6-t-butylindane (1252 grams, 62% recovery) and 878 grams of 4-acetyl-6-t-butyl-1, 1-dimethylindane (90% yield based on acetyl chloride, 95% yield based on consumed 1, 1-dimethyl-6-t-butylindane).

BAD ORIGINAL

Example II

5-Acetyl-1, 1, 2, 3, 3, 6-hexamethyl-1H-indane

Reaction:

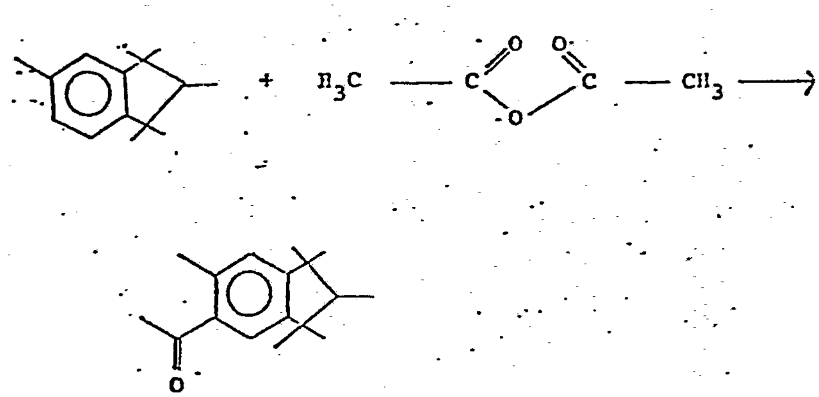

A solution of 1,1,2,3,3,6-hexamethyl-1H-indane (566 grams) and acetic anhydride (306 grams) is added over a 4 hour period at -10$^{o}$C to a slurry of aluminium chloride (534 grams), 1,1,2,3,3,6-hexamethyl-1H-indane (1050 grams) and isooctane (404 grams). The reaction is washed up as in Example I followed by washing with 1 litre of sodium hydroxide (5% aqueous solution). Distillation affords 395 grams of 5-acetyl-1,1,2,3,3,6-hexamethyl-1H-indane (54% yield based on charged $AlCl_3$) and 1192 grams of recovered 1, 1, 2, 3, 3, 6-hexamethyl-1H-indane.

Example III

1,1, 4, 4-Tetramethyl-6-ethyl-7-acetyl-1, 2, 3, 4-tetrahydronaphthalene

Reaction:

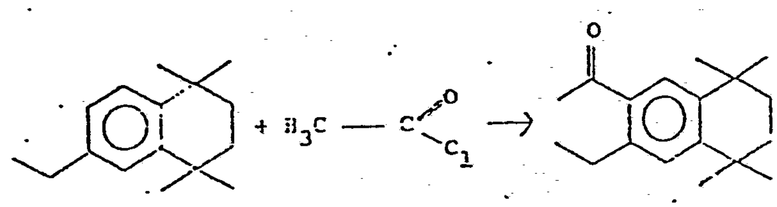

A solution of 1,1,4,4-tetramethyl-6-ethyl-1,2,3,4-tetrahydronaphthalene (528 grams) and acetyl chloride (234 grams) is added over a 3 1/2 hour period at 70°C to a well stirred slurry of aluminium chloride (534 grams) 1,1,4,4-tetramethyl-6-ethyl-1,2,3,4-tetrahydronaphthalane (1200 grams) and isooctane (432 grams). The reaction is worked up as in Example II to afford 689 grams of 1,1,4,4-tetramethyl-6-ethyl-7-acetyl-1,2,3,4-tetrahydronaphthalene (89% based on consumed 1,1,4,4-tetramethyl-6-ethyl-1,2,3,4-tetrahydronaphthalene) and 1106 grams of recovered 1,1,4,4-tetramethyl-6-ethyl-1, 2, 3, 4-tetrahydronaphthalene.

## CLAIMS

1. A process for preparing acyl benzene derivatives having the structures:

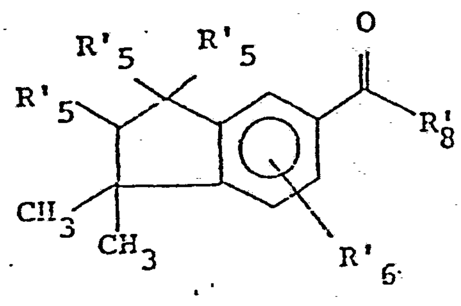

or

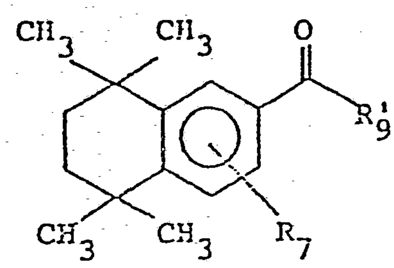

wherein $R'_5$ is hydrogen or methyl; $R_6$ and $R'_7$ are the same or different hydrogen or $C_1$-$C_3$ lower alkyl; $R'_8$ and $R'_9$ are $C_1$ to $C_3$ and are the same or different lower alkyl, comprising the steps of reacting an indane or tetrahydronaphthalene having the structures:

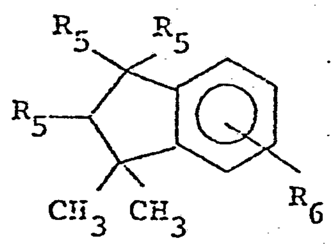

or

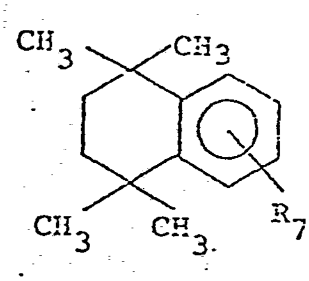

with an acid derivative having the structure:

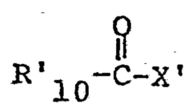

wherein $R'_{10}$ is $C_1$-$C_3$ lower alkyl and X' is -0-C-$R'_{10}$ or chloride, bromide or iodide, in the presence of a saturated hydrocarbon or hydrocarbon mixture comprising the groups $C_5$ to $C_{10}$ alkanes at a temperature between -20° and -5°C in the presence of an aluminium chloride catalyst; the ratio of the aliphatic saturated hydrocarbon to the indane or tetrahydronaphthalene derivative being from 1:10 up to 10:1; the mole ratio of aluminium chloride to acid derivative being 1:2 to 2:1; the mole ratio of indane or tetrahydronaphthalene derivative to acid derivative being from 1:1 up to 10:1.

BAD ORIGINAL

2. A process according to claim 1 wherein the ratio of hydrocarbon reactant to acid derivative is 2:1.

3. A process according to claim 1 wherein the saturated hydrocarbon or hydrocarbon mixture comprises one or more of n-hexane, n-octane and 2, 2, 4-trimethyl pentane.

4. A process according to claim 1 wherein the ratio of saturated hydrocarbon to hydrocarbon reactant is from 2:10 to 3:10.

5. A process of producing a synthetic musk compound substantially as described in any one of the foregoing Examples.

6. A product whenever produced by the process of Claim 1, 2, 3, 4 or 5.

BAD ORIGINAL